# FASCICULE DE BREVET EUROPEEN

(11) **EP 2 766 003 B1**
(45) Date de publication et mention de la délivrance du brevet: **13.12.2017**
(21) Numéro de dépôt: 12769682.1
(22) Date de dépôt: 10.10.2012
(51) Int. Cl.: A61K 31/13, A61P 25/02

(54) **COMPOSITION POUR LE TRAITEMENT PROPHYLACTIQUE DE LA DOULEUR NEUROPATHIQUE**
ZUSAMMENSETZUNG ZUR VORBEUGUNG VON NEUROPATHISCHEM SCHMERZ
COMPOSITION FOR PREVENTION OF NEUROPATHIC PAIN

(30) Priorité: 11.10.2011 FR 1159147
(43) Date de publication de la demande: 20.08.2014
(73) Titulaire: Université d'Auvergne, 63000 Clermont-Ferrand (FR)
(72) Inventeur: PICKERING, Gisèle, F-63000 Clermont-Ferrand (FR)
(74) Mandataire: Vidon Brevets & Stratégie
(86) Numéro de dépôt international: PCT/EP2012/070043
(87) Numéro de publication internationale: WO 2013/053746

(56) Documents cités:
- WO-A1-02/100434
- EISENBERG ELON ET AL: "The clinically tested N-methyl-D-aspartate receptor antagonist memantine blocks and reverses thermal hyperalgesia in a rat model of painful mononeuropathy", NEUROSCIENCE LETTERS, vol. 187, no. 1, 1995, pages 17-20, XP002674418, ISSN: 0304-3940
- WILSON ET AL: "NMDA receptor antagonist treatment at the time of nerve injury prevents injury-induced changes in spinal NR1 and NR2B subunit expression and increases the sensitivity of residual pain behaviours to subsequently administered NMDA receptor antagonists", PAIN, ELSEVIER SCIENCE PUBLISHERS, AMSTERDAM, NL, vol. 117, no. 3, 1 octobre 2005 (2005-10-01), pages 421-432, XP005092901, ISSN: 0304-3959, DOI: 10.1016/J.PAIN.2005.07.005
- POZZI A ET AL: "Prevention of central sensitization and pain by N-methyl-D-aspartate receptor antagonists", JOURNAL OF THE AMERICAN VETERINARY MEDICAL ASSOCIATION, AMERICAN VETERINARY MEDICAL ASSOCIATION, US, vol. 228, no. 1, 1 janvier 2006 (2006-01-01), pages 53-60, XP009158598, ISSN: 0003-1488
- KIEFER RALPH T ET AL: "Prevention of Phantom Limb Pain and Cortical Reorganization after Traumatic Upper Limb Amputations.", ANESTHESIOLOGY ABSTRACTS OF SCIENTIFIC PAPERS ANNUAL MEETING, no. 2001, 2002, XP002674419, & 2001 ANNUAL MEETING OF THE AMERICAN SOCIETY OF ANESTHESIOLOGISTS; NEW ORLEANS, LA, USA; OCTOBER 13-17, 2001
- MEDVEDEV IVAN O ET AL: "Effects of low-affinity NMDA receptor channel blockers in two rat models of chronic pain", NEUROPHARMACOLOGY, vol. 47, no. 2, août 2004 (2004-08), pages 175-183, XP002674420, ISSN: 0028-3908

## Description

### 1. Domaine de l'invention

Le domaine de l'invention est celui des compositions pharmaceutiques utilisables pour le traitement de la douleur.

Plus précisément, l'invention concerne une composition comprenant de la mémantine pour la prévention de la douleur neuropathique post-chirurgicale.

### 2. Art antérieur

La douleur neuropathique est une douleur neurogène. Les symptômes sont nombreux : sensations d'élancements, de décharges électriques, sensations de brûlures ou de froid douloureux dans les territoires des nerfs atteints, hyperalgésie, allodynie... La douleur neuropathique est une conséquence directe d'une lésion ou d'une pathologie affectant le système sensoriel : neuropathie induite par un diabète, un traumatisme, un acte chirurgical, une infection au VIH ou post-herpétique, douleur du membre fantôme suite à une amputation, ...

Plusieurs traitements ont été proposés pour prendre en charge la douleur neuropathique. Ils n'ont toutefois pas toujours démontré une bonne efficacité et ce, en fonction des patients et de l'étiologie de la douleur.

De nombreuses études ont démontré l'implication du récepteur N-Methyl-D-Aspartate (récepteur NMDA). Le récepteur NMDA est un récepteur ionotrope activé par le glutamate. Il se présente sous la forme d'un canal ouvert, perméable aux ions calcium et aux cations monovalents tels que les ions sodium et potassium. En condition physiologique de repos, l'ouverture du canal est obturée par un ion magnésium. L'influx d'ions calcium ou sodium, suite à une excitation des nerfs, déplace l'ion magnésium et permet la sortie des ions potassium. Sur le plan de la structure, ce canal est composé de quatre sous-unités : deux sous-unités NR1 et deux sous-unités NR2 (NR2A, NR2B, NR2C ou NR2D). Les sous-unités NR1 sont constitutives et toujours présentes. Les sous-unités NR2, quant à elles, spécifient les propriétés du canal comme la sensibilité au glutamate et la perméabilité aux ions calcium.

Le récepteur NMDA est ubiquitaire et est impliqué dans de nombreuses fonctions: apprentissage et mémoire, plasticité des synapses, mécanismes de la douleur. Un grand espoir a été placé dans l'utilisation des antagonistes pour inhiber l'activation de ce récepteur, et ainsi traiter la douleur neuropathique. Parmi les antagonistes utilisés, on peut notamment citer la kétamine, le dextromethorphan ou le MK-801. Bien qu'efficaces, ces molécules antagonistes du récepteur NMDA présentent une toxicité qui se manifeste notamment par des troubles psychodysleptiques ou des hallucinations.

La mémantine (1-amino-3,5-dimethyl-adamantane) est un dérivé de l'amantadine. Cette molécule a d'abord été utilisée, vers la fin des années 70, comme traitement de la maladie de Parkinson, puis comme traitement dans la maladie d'Alzheimer. Certaines études ont mis en évidence son effet positif dans le développement de la douleur neuropathique sur les rats chez lesquels des douleurs neuropathiques ont été induites grâce à la ligature du nerf sciatique ou l'injection de formaline (Eisenberg et al, Neurosciences Letters 187, 1995 ; Carlton et al., Neuroscience Letters, 198, 1995). Dans ces études, la mémantine a été administrée pendant ou après l'opération. Un effet bénéfique a également été rapportée chez des patients ayant subi une amputation, à condition d'administrer la mémantine juste après l'opération (Buvanendran et al, International Anesthesia Research Society, 107 (4), 2008).

Toutefois d'autres études viennent limiter l'enthousiasme entourant ce constat. Dans un article paru en 2000, une équipe danoise a démontré que la mémantine est inefficace pour soulager des patients ayant subi l'amputation d'un membre ou présentant des lésions nerveuses suite à une chirurgie (Nikolajsen et al., International Anesthesia Research Society, 91, 2000).

Enfin, une récente revue des études cliniques menées chez l'homme et utilisant de nombreux antagonistes du récepteur NMDA a démontré que les molécules, les doses utilisées et les résultats étaient considérablement variés. Les auteurs de cette revue en déduisent qu'aucune réelle conclusion concernant l'efficacité des antagonistes du récepteur NMDA sur la douleur neuropathique ne peut être établie (Collins et al, Pain Medicine, 11, 2010).

Dans la mesure où ce type de douleur affecte un grand nombre de patients, qu'elle est source de nombreuses complications pour le patient, aussi bien physiques que psychologiques, il est nécessaire d'explorer de nouvelles voies pour prévenir, ou du moins traiter, l'apparition de la douleur neuropathique.

### 3. Objectifs de l'invention

L'invention a notamment pour objectif de pallier ces inconvénients de l'art antérieur.

Plus précisément, un objectif de l'invention est de fournir, dans au moins un mode de réalisation, une composition permettant de prévenir, ou à tout le moins, de limiter l'apparition de la douleur neuropathique.

L'invention a encore pour objectif de proposer, dans au moins un mode de réalisation, une posologie permettant de prévenir, ou à tout le moins, de limiter l'apparition de la douleur neuropathique.

### 4. Exposé de l'invention

Ces objectifs, ainsi que d'autres qui apparaîtront par la suite, sont atteints à l'aide d'une composition analgésique.

Selon l'invention, une telle composition est inhibitrice de la phosphorylation du résidu Tyrosine 1472 de la sous-unité NR2B du récepteur NMDA, ladite composition étant destinée à prévenir le développement de la douleur neuropathique post-opératoire chez un patient humain devant subir une opération.

Il a été découvert d'une manière surprenante que la phosphorylation des résidus Tyrosine en position 1472 et 1336 de la sous-unité NR2B du récepteur NMDA était un phénomène clairement corrélé au développement de la douleur neuropathique. Plus particulièrement, la phosphorylation du résidu Tyrosine 1472 est concomitante au phénomène d'hyperalgésie. L'hyperalgésie se caractérise par la sensation de douleur accrue à un stimulus en soi douloureux. Elle est une conséquence commune à la douleur neuropathique, en particulier dans les neuropathies induites par le diabète. Bien que les mécanismes moléculaires de la douleur neuropathique dans le diabète ne soient pas encore clairement identifiés, il a été découvert que l'inhibition des protéines kinases Src et FAK, responsables de la phosphorylation des résidus Tyrosine 1472 et 1336 de la sous-unité NR2B, résultent en une diminution du phénomène d'hyperalgésie.

Selon l'invention, une telle composition comprend de la mémantine. La mémantine inhibe de manière significative la phosphorylation du résidu Tyrosine en position 1472 (notée pTyr¹⁴⁷²) de la sous-unité NR2B du récepteur NMDA. Elle est de plus relativement bien tolérée par les patients. Un traitement chronique par voie intrathécale avec un inhibiteur de la protéine kinase Src, le PP2 (de formule 4 - amino- 5 - (4 - chlorophenyl) - 7 - (dimethylethyl) pyrazolo [3,4 - d] pyrimidine) ou un oligodeoxynucléotide antisens dirigée contre la protéine FAK, abolit l'hyperalgie mécanique induite par le diabète. Ce traitement diminue de plus la phosphorylation du résidu Tyr¹⁴⁷², suggérant un rôle important de Src et FAK dans ce contexte douloureux.

Dans un mode de réalisation, une telle composition comprend entre 4 mg et 17 mg de mémantine pure.

Dans un autre mode de réalisation avantageux, la composition selon l'invention comprend entre 5 mg et 20 mg de chlorydrate de mémantine.

Selon l'invention, une telle composition se présente sous forme de comprimé pelliculé ou de solution buvable. Ces formes galéniques présentent en effet l'avantage d'être facilement administrables. La composition peut ainsi être prise par le patient seul, à son domicile, et ne nécessite pas l'intervention de personnel médical.

Dans un autre mode de réalisation avantageux, la composition peut se présenter sous forme injectable et peut être administrée au patient, en continu ou sous forme de bolus, grâce à une perfusion de mémantine ou de chlorhydrate de mémantine liquide.

L'invention a encore pour objet une composition analgésique contenant de la mémantine. Selon l'invention, une telle composition est administrée quotidiennement à un patient humain devant subir une opération chirurgicale, sur une période allant de un à plusieurs jours avant ladite opération chirurgicale, pour prévenir le développement de la douleur neuropathique post-opératoire chez ce même patient.

Bien que la littérature citée en la matière ne cite que l'utilisation de la mémantine en peropératoire ou postopératoire, c'est-à-dire pendant ou après l'opération, les inventeurs ont découvert de manière étonnante que l'administration quotidienne de mémantine, sur une période allant de un à plusieurs jours avant l'opération, permet de limiter de manière efficace l'apparition de la douleur neuropathique. Des études chez le rat ont démontré que l'administration de mémantine, pendant ou après l'opération, limite l'apparition des symptômes de la douleur neuropathique, mais seulement de manière dose-dépendante et uniquement pour un temps déterminé au bout duquel les symptômes réapparaissent (Eisenberg et al, Neurosciences Letters 187, 1995). Bien que ce document utilise les termes de « prophylaxie » et de « prévention », il s'agit d'une administration pendant l'opération, c'est-à-dire une administration peropératoire de la mémantine et non une administration en amont de l'opération chirurgicale au sens de l'invention.

Au contraire, les inventeurs ont démontré que l'administration prophylactique de mémantine, c'est-à-dire entre un et plusieurs jours avant l'opération et non uniquement pendant ou après ladite opération, permettait d'éviter l'apparition des symptômes liés à la douleur neuropathique, à savoir hyperalgésie, allodynie, troubles mécaniques ainsi que les troubles cognitifs habituellement observés.

Le terme de « prophylaxie » dans la littérature est souvent utilisé pour désigner l'administration peropératoire, c'est-à-dire pendant l'opération, au moment de l'anesthésie associée à l'opération, ou juste après la réalisation de l'acte chirurgical. Au sens de l'invention et dans la description qui va suivre, le terme de « prophylaxie » est utilisé pour désigner l'administration au patient de la composition selon l'invention entre un et plusieurs jours avant l'acte chirurgical. En d'autres termes, et au sens de l'invention, une administration préventive ou prophylactique consiste à administrer au patient devant être opéré la composition selon l'invention en amont de son opération. Le patient reçoit donc une dose quotidienne de la composition selon l'invention la veille, voire pendant les jours qui précèdent son opération.

Jusqu'à présent, la pratique clinique consistait à administrer la mémantine au moment de l'anesthésie du patient, soit pendant l'opération.

Cette distinction est importante car c'est grâce à cette administration réalisée entre un et plusieurs jours en amont de l'opération que la désensibilisation du récepteur NMDA sera effective et que le patient ne développera pas ou prou de symptômes liés à la douleur neuropathique.

Le patient gagne donc en qualité de vie et sa consommation d'antalgiques en est considérablement diminuée. Cela permet également au patient de récupérer plus rapidement de sa chirurgie, les douleurs mécaniques et sensorielles étant diminuées. Les conséquences néfastes d'une telle douleur chronique et à la limite du supportable sur la santé physique et mentale du patient en sont donc considérablement limitées.

On entend par administration préopératoire, une administration réalisée la veille ou plusieurs jours précédent l'opération.

On entend par administration peropératoire une administration réalisée pendant l'opération ou au moment de l'anesthésie liée à l'opération.

Dans un mode de réalisation avantageux, la composition selon l'invention est administrée pendant une période comprise entre 1 jour et 42 jours avant ladite opération chirurgicale.

De préférence, la composition selon l'invention est administrée pendant une période comprise entre 1 jour et 28 jours avant ladite opération chirurgicale.

La composition analgésique remplit son rôle de prophylaxie de la douleur lorsqu'elle est administrée quatre semaines avant l'opération.

De manière encore plus préférée, la composition selon l'invention est administrée pendant une période comprise entre 7 jours et 14 jours précédents ladite opération chirurgicale.

Une durée d'administration comprise entre 1 et 2 semaines avant l'opération permet à la fois de ne pas sensibiliser les récepteurs NMDA du patient pour éviter, ou à tout le moins, limiter le développement de la douleur neuropathique, mais elle permet également de limiter la prise de médicaments dans le temps ce qui est toujours positif pour un patient.

Dans un mode de réalisation avantageux, l'administration préopératoire d'une composition selon l'invention se fait par paliers successifs de doses croissantes.

Le récepteur NMDA étant impliqué dans de nombreux mécanismes du système nerveux central, il est nécessaire d'habituer l'organisme du patient de manière progressive. Cette méthode de titration de la composition selon l'invention a pour but de déterminer la dose maximale permettant une efficacité satisfaisante chez le patient, tout en évitant les effets indésirables liés à tout médicament agissant sur le système nerveux central. Au sens de l'invention, la composition est administrée par paliers d'une semaine, en augmentant la dose de 5 mg/jour à chaque palier.

De préférence, la composition selon l'invention est administrée sous forme de chlorhydrate de mémantine à une dose comprise entre 5 mg/jour et 20 mg/jour.

Plus précisément, la composition selon l'invention est administrée, pendant une durée de 28 jours précédents l'opération, à une dose de :
- 5 mg/jour de chlorhydrate de mémantine pendant la première semaine,
- 10 mg/jour de chlorhydrate de mémantine pendant la deuxième semaine,
- 15 mg/jour de chlorhydrate de mémantine pendant la troisième semaine
- 20 mg/jour de chlorhydrate de mémantine pendant la quatrième semaine.

Au sens de l'invention, la dose de chlorhydrate de mémantine à administrer au patient peut aller jusqu'à 30 mg/jour. En ce cas, la composition selon l'invention sera administrée pendant une durée de 42 jours avant l'opération du patient, afin de respecter les paliers de progression de 5 mg par semaine.

Il est également envisageable, au sens de l'invention, de prescrire des doses supérieures allant jusqu'à 55 mg/jour de mémantine à un patient, à condition de respecter la progression de 5 mg par semaine.

À titre de précision, la dose de :
- 5 mg de chlorhydrate de mémantine équivaut à une dose de 4,15 mg de mémantine pure,
- 10 mg de chlorhydrate de mémantine équivaut à une dose de 8,31 mg de mémantine pure
- 15 mg de chlorhydrate de mémantine équivaut à une dose de 12,46 mg de mémantine pure
- 20 mg de chlorhydrate de mémantine équivaut à une dose de 16,62 mg de mémantine pure
- 25 mg de chlorhydrate de mémantine équivaut à une dose de 20,77 mg de mémantine pure
- 30 mg de chlorhydrate de mémantine équivaut à une dose de 24,93 mg de mémantine pure.

Ces doses sont les doses maximales testées cliniquement et sont bien supportées et tolérées par les patients. Elles permettent d'allier efficacité d'action sans altérer les capacités mécaniques et cognitives du patient (Sang et al., 2002).

Dans un autre mode de réalisation de l'invention, la composition selon l'invention est administrée sous forme de mémantine pure à une dose comprise entre 4,15 mg/ jour et 24,93 mg/ jour.

Dans un autre mode de réalisation davantage préféré, la composition selon l'invention est, en outre, administrée en postopératoire.

Avantageusement, la composition selon l'invention est également administrée pendant l'opération, à une dose correspondant au palier atteint en préopératoire.

L'administration au patient de la composition selon l'invention après l'opération permet de prolonger son action bénéfique en maintenant les récepteurs NMDA dans un état désactivé suite au traumatisme de l'opération.

De préférence, l'administration en post-opératoire de la composition selon l'invention se fait à dose constante.

En effet, il est nécessaire, pour que l'effet préventif de la composition soit pleinement réalisé, que le plateau de titration soit atteint avant l'opération. L'administration de mémantine se fait alors à dose constante, ladite dose correspondant au plateau atteint.

De manière avantageuse, la composition selon l'invention est alors administrée quotidiennement et en postopératoire à une dose constante comprise entre 15 mg/ jour et 30 mg/jour.

De préférence, la composition selon l'invention est en outre administrée en postopératoire pendant une durée comprise entre 1 et 16 jours.

Selon l'invention, l'administration postopératoire de la composition selon l'invention pendant une durée maximale de 16 jours suffit à maintenir les effets bénéfiques de la prophylaxie et à limiter l'apparition de la douleur neuropathique et des symptômes associés (hyperalgésie, allodynie, troubles mécaniques...). Il est toutefois entendu que l'administration postopératoire de mémantine peut être prolongée au-delà de 16 jours, si la douleur du patient est persistante, et continuer jusqu'à la disparition complète des douleurs résiduelles.

Dans un mode de réalisation avantageux, une telle composition prophylactique est administrée par voie orale, sous forme de comprimé pelliculé ou de solution buvable.

Ce mode d'administration simplifie le suivi du traitement par le patient à son domicile. Les comprimés pelliculés peuvent être en outre à libération prolongée.

De préférence, la composition selon l'invention caractérisée en ce qu'elle est administrée au patient le matin.

Elle peut être indépendamment prise pendant ou en dehors du repas. Il est toutefois important que la prise de mémantine soit effectuée à heure régulière afin de maintenir les récepteurs NMDA dans un état désactivé.

Dans un autre mode de réalisation de l'invention, la composition selon l'invention est administrée par perfusion à une concentration comprise entre 5 mg/jour/kg et 20 mg/jour/kg.

Selon l'invention, la composition prophylactique est destinée à être administrée à un patient devant subir une chirurgie pouvant entraîner des douleurs neuropathiques, ladite chirurgie étant choisie parmi l'ablation d'un membre, d'une partie d'un membre, d'un organe ou d'une partie d'un organe.

L'amputation d'un membre ou l'ablation d'une partie d'un organe est systématiquement accompagnée de l'élimination d'une partie des nerfs associés à ce membre ou cette partie d'un organe. Il en résulte un fort traumatisme pour l'organisme du patient qui se manifeste par une douleur intense et chronique. On connaît également le phénomène de la douleur du membre fantôme, qui est un phénomène qui concerne 90% à 98% des les patients amputés. Elle est dénommée ainsi car le patient ressent une douleur intense dans ce qui lui semble être son membre amputé, bien qu'il sache que cette douleur n'a rien de réel. Cela provient d'une stimulation constante et intense des nerfs liés à un territoire anatomique particulier, ce territoire étant associé à un membre ou à un organe dans la cartographie somato-sensorielle du cerveau.

Administrer une composition selon l'invention de manière prophylactique, à savoir pendant une durée comprise entre 1 et 42 jours avant l'opération, de préférence pendant une durée comprise entre 1 jour et 28 jours avant l'opération et de manière encore plus préférée pendant une durée comprise entre 7 et 14 jours avant l'opération, permet d'éviter, ou du moins de limiter grandement, l'apparition de cette douleur neuropathique. Les patients concernés par ce type de douleurs sont extrêmement nombreux : amputation d'un membre ou d'une partie d'un organe suite à une ischémie du membre ou une infection, amputation d'une partie d'un organe pour éliminer une masse tumorale comme c'est le cas dans les cancers du sein, de l'os, du foie, du poumon, du muscle, du rein, ...

L'invention a encore pour objet une méthode pour prévenir la douleur neuropathique post-opératoire, chez un patient humain devant subir une opération chirurgicale, par administration prophylactique de mémantine.

La méthode selon l'invention permet d'éviter l'apparition des symptômes de la douleur neuropathique, ou du moins de limiter leur intensité grâce à l'administration préventive et quotidienne de mémantine à un patient. Par administration préventive, on entend que la mémantine se doit d'être administrée au patient avant son opération et pas seulement pendant et juste après l'opération. Cette administration préopératoire permet de ne pas sensibiliser les récepteurs NMDA impliqués dans le développement de la douleur neuropathique et l'apparition des symptômes associés tels que l'hyperalgésie, l'allodynie et les troubles mécaniques.

Dans un mode de réalisation avantageux, la méthode selon l'invention prévoit d'administrer la mémantine quotidiennement pendant une période allant de 1 à 28 jours avant l'opération que doit subir ledit patient humain.

De préférence, la méthode selon l'invention prévoit que l'administration préopératoire de mémantine se fait par paliers successifs de doses croissantes.

Cette administration par paliers successifs de doses croissantes de mémantine, que l'on appelle également titration, permet d'habituer l'organisme du patient graduellement à l'action inhibitrice de la mémantine sur les récepteurs NMDA. Ces récepteurs étant ubiquitaires et intervenant dans de nombreux processus du système nerveux central, il est conseillé d'avoir une progression posologique.

Selon l'invention, cette titration se fait par palier d'une semaine, la dose de mémantine administrée quotidiennement au patient augmentant de 5 mg par semaine.

De préférence, la méthode selon l'invention prévoit que la mémantine est administrée sous forme de chlorhydrate de mémantine, à une dose comprise entre 5 mg/jour et 20 mg/jour. Cette dose peut être augmentée jusqu'à 30 mg/jour.

Dans un autre mode de réalisation avantageux de l'invention, la méthode selon l'invention prévoit que la mémantine peut être administrée sous forme pure à une dose comprise entre 4,15 mg/jour et 24,93 mg/jour.

La méthode selon l'invention prévoit en outre que la mémantine peut être administrée en peropératoire et en postopératoire, c'est-à-dire que la mémantine est administrée pendant et après l'opération.

Cette étape permet de maintenir les récepteurs NMDA dans un état inhibé malgré le traumatisme de l'opération et des lésions infligées aux terminaisons nerveuses.

De préférence, la méthode selon l'invention prévoit que l'administration peropératoire et postopératoire de mémantine se fait à dose constante.

En ce cas, la dose utilisée correspond à la dose maximale à laquelle la titration est parvenue. Par exemple, si la titration s'est effectuée sur 28 jours pour parvenir à une dose maximale de 20 mg/jour de chlorhydrate de mémantine, la dose de mémantine utilisée en peropératoire et/ou postopératoire sera de 20 mg/jour.

Dans un mode de réalisation avantageux, l'étape d'administration postopératoire de mémantine se déroule sur une durée comprise entre 1 et 16 jours après l'opération.

Dans un autre mode de réalisation, la méthode selon l'invention prévoit que la mémantine est administrée le matin.

Dans un mode de réalisation préférée, la méthode de prévention et de traitement de la douleur neuropathique selon l'invention s'applique à des patients devant subir l'ablation d'un membre, d'une partie d'un membre, d'un organe ou d'une partie d'un organe.

### 5. Liste des figures

D'autres caractéristiques et avantages de l'invention apparaîtront plus clairement à la lecture de la description suivante et des dessins annexés, parmi lesquels :
- la figure 1 présente un graphique représentant les effets de l'administration postopératoire de mémantine sur l'hyperalgésie mécanique chez le rat.
- la figure 2 illustre, en comparaison avec la figure 1, les effets de l'administration de la mémantine en préopératoire et postopératoire sur l'hyperalgésie mécanique, chez le rat.
- la figure 3 présente un graphique représentant les effets de l'administration postopératoire de mémantine sur l'allodynie mécanique chez le rat.
- la figure 4 illustre, en comparaison avec la figure 3, les effets de l'administration de la mémantine en préopératoire et postopératoire sur l'allodynie mécanique chez le rat sous forme de graphique.
- la figure 5 est un graphique présente les effets de l'administration prophylactique de la mémantine sur la mémoire spatiale chez le rat.
- la figure 6 est une photographie d'un gel de Western Blot présentant les effets de la mémantine sur la phosphorylation du résidu Tyrosine 1472 de la sous-unité NR2B du récepteur NMDA chez le rat.
- la figure 7 est un graphique présentant les résultats d'un essai clinique de phase 2 chez 15 patientes ayant reçu soit une composition placebo, soit la composition selon l'invention.

### 6. Description d'un mode de réalisation de l'invention

Le principe général de l'invention repose sur l'administration préventive, c'est-à-dire un à plusieurs jours avant l'opération programmée d'un patient, d'un inhibiteur des récepteurs NMDA. Plus particulièrement, cet inhibiteur peut être un inhibiteur de la phosphorylation du résidu Tyrosine 1336 ou 1472 de la sous unité NR2B du récepteur NMDA. Cet inhibiteur peut également être un antagoniste du récepteur NMDA, telle que la mémantine. Cette administration préopératoire, c'est à dire entre un et plusieurs jours avant l'opération, de mémantine permet de ne pas sensibiliser les récepteurs NMDA, impliqués dans le développement de la douleur neuropathique. Cette non sensibilisation précoce permet non seulement de limiter l'intensité des symptômes associés à la douleur neuropathique, mais encore d'éviter leur survenue.

### 6.1 Etude de l'effet de la mémantine sur la douleur neuropathique induite par la ligature du nerf spinal chez le rat.

Les effets de l'administration de manière prolongée et préventive de mémantine sont d'abord étudiés chez le rat. Le modèle utilisé est celui de la ligature du nerf spinal L5 (ci-après dénommée SNL pour Spinal Nerve Ligation) chez le rat. La survenue de la douleur neuropathique est appréciée en fonction des critères suivants :
- le comportement du rat suite à la ligature du nerf spinal (SNL),
- la cognition, et
- les évènements moléculaires dont l'expression de la tyrosine 1472 phosphorylée de la sous-unité NR2B du récepteur NMDA au niveau spinal.

La ligature du nerf spinal chez le rat provoque une neuropathie douloureuse accompagnée d'hyperalgésie et d'allodynie, symptômes fréquemment décrits chez les patients atteints de douleur neuropathique.

L'hyperalgésie, ou hyperalgie, correspond à une sensation de douleur accrue ressentie suite à un stimulus douloureux. L'allodynie correspond à une sensation de douleur accrue ressentie suite à un stimulus normalement indolore chez un individu bien portant.

L'étude se base sur la comparaison de deux principaux lots d'animaux. Le premier groupe est un groupe contrôle. Les rats du groupe contrôle, désignés sous le terme « Sham » dans les graphiques, ne subissent pas d'opération. Ils permettent d'évaluer le rôle des substances injectées chez l'individu, sans qu'une douleur neuropathique n'intervienne. Parmi ce groupe, sont réalisés deux sous groupes :
- un sous groupe traité par le véhicule qui est une solution saline de NaCl 0.9 % massique, à une dose de 1 mL/kg/rat/jour, par injection intrapéritonéale ; et
- un sous groupe traité par de la mémantine à une dose de 20 mg/kg/rat/jour, par injection intrapéritonéale.

Le second lot d'animaux comprend les rats devant subir une ligature du nerf spinal, et donc, devant développer une douleur neuropathique. Ils sont signalés dans les graphiques par le terme SNL. De même que pour le groupe contrôle, ce groupe est subdivisé en deux sous-groupes :
- un sous groupe traité par le véhicule qui est une solution saline de NaCl 0.9 % massique, à une dose de 1 mL/kg/rat/jour, par injection intrapéritonéale ; et
- un sous groupe traité par de la mémantine à une dose de 20 mg/kg/rat/jour, par injection intrapéritonéale.

Enfin, parmi ces groupes et sous groupes, certains rats reçoivent leur injection quotidienne de solution saline ou de mémantine pendant 4 jours avant et pendant 3 jours après l'opération, et d'autres rats ne reçoivent leur injection quotidienne que pendant les 3 jours qui suivent l'opération. Le nombre de rats dans chaque groupe et dans chaque condition est indiqué entre parenthèses sur les figures.

### 6.1.1 Ligature du nerf spinal (SNL)

Brièvement, la ligature du nerf spinal chez le rat se fait selon les étapes suivantes.
1. Les rats reçoivent une injection intrapéritonéale de pentobarbital (6 %, 1 ml/kg). Les rats n'étant pas anesthésiés à l'issue de cette injection seront ré-anesthésiés le lendemain à une dose de 1.2 ml/kg. La partie lombaire du dos des rats est tondue puis désinfectée (Bétadine et alcool à 70°C).
2. La peau est incisée sur environ 2 cm de part et d'autre de L5, environ 0.5 cm à gauche de la colonne vertébrale. Le muscle paraspinal gauche est disséqué afin de pouvoir dégager la partie latérale de la colonne vertébrale. Le processus transverse L5 est mis à nu, puis est retiré à l'aide d'une gouge, dégageant ainsi le nerf L5. Ce dernier est isolé sur une micropipette en verre de très petit diamètre.
3. À l'aide d'une micropipette en verre et d'un noeud coulant, un fil de soie est passé sous le nerf L5 et noué autour du nerf ; les 2 extrémités du fil sont coupées à environ 3 mm du noeud.
4. Une fois la ligature des nerfs effectuée, le muscle et la peau sont recousus. La cicatrice est désinfectée à la Bétadine. Une solution antibiotique (Négérol®, Céva Santé Animale, 150 ml/142 g) est vaporisée sur la cicatrice. Une solution de NaCl 0.9 % est injectée par voie sous-cutanée (s.c., 10 ml/kg) pour réhydrater le rat.
5. Les rats sont isolés dans une cage sous une lampe jusqu'à leur réveil. Des pellets d'aliments sont placés dans la sciure. Puis, un anti-inflammatoire non-stéroïdien, le Meloxicam (Mobic®, 2 mg/kg) est administré par voie sous-cutanée pendant deux jours.
6. Les animaux sont observés quotidiennement afin de dépister tout comportement inhabituel. Les anomalies de la sensation douloureuse apparaissent entre 3 et 10 jours après la chirurgie et persistent pendant au moins 28 jours.
7. Sept à quatorze jours après la chirurgie, les rats neuropathiques hyperalgésiques ou allodyniques sont sélectionnés d'après leur réponse à une stimulation nociceptive ou non nociceptive évaluée à l'aide de tests appropriés (voir le paragraphe 6.1.2). Seuls les animaux dont les seuils de nociception sont réduits d'au moins 15% par rapport aux seuils mesurés avant la chirurgie ou ceux pour lesquels l'application d'une stimulation non douloureuse évoque une réponse douloureuse sont respectivement considérés comme hyperalgésiques ou allodyniques et inclus dans l'étude. Les rats non hyperalgésiques ou non allodyniques (environ 1 à 5% des rats opérés) sont écartés de l'étude.

### 6.1.2 Tests comportementaux et cognitifs utilisés pour sélectionner les rats présentant une douleur neuropathique :

### Test de pression de la patte : hyperalgésie mécanique:

Les rats sont soumis au test de pression de la patte (Randall & Selitto, 1957). Le seuil de nociception, la vocalisation ou la réaction de lutte, est mesuré à l'aide un analgésimètre Ugo Basile (Bioseb®). Celui-ci exerce, grâce à un cône placé sur la patte postérieure gauche du rat, une pression croissante par le déplacement d'un poids le long d'une échelle graduée. La stimulation est immédiatement arrêtée dès la réaction de l'animal. La distance parcourue par le poids, en centimètres, est multipliée par 30 pour obtenir le seuil douloureux en grammes. La pression maximale exercée ou « cut-off » est de 450 g. Toute diminution du seuil de vocalisation est signe d'une hyperalgésie.

### Test de Von Frey : allodynie mécanique:

Huit filaments de Von Frey (Chaplan et al., 1994) sont utilisés dans cette étude, allant de 1, 4 grammes à 26 grammes. Les rats sont placés sur le support pendant 15 à 20 minutes pour habituation. Les poils sont appliqués perpendiculairement à la surface plantaire du rat au niveau des coussinets, à commencer par le filament de 6 grammes. La taille des filaments suivants sera déterminée par la réponse du rat, selon la méthode de Chaplan (1994). Si le rat réagit au stimulus, le filament suivant sera celui situé immédiatement en dessous dans la gamme, et vice versa. L'expérience continue jusqu'à ce que 4 filaments aient été appliqués après le premier changement de comportement de réponse du rat. Les rats reçoivent ainsi entre 5 et 9 stimuli au total. La réponse des rats est exprimée par le seuil de réponse 50% d'après la formule de Dixon (1980).

### Test Y-Maze : Mémoire spatiale

Le rat est placé au centre d'une enceinte à trois bras mesurant entre 40 cm de long et 4 cm de large. Le rat circule ainsi librement entre les différents bras pendant 5 minutes et le nombre d'entrées est alors comptabilisé entre chacun des bras. Ce test non invasif est ainsi basé sur le comportement exploratoire de l'animal et sur la capacité cognitive de ce dernier (Dellu et al., 1992).

L'étude se déroule selon la chronologie suivante :
- J-6 : test de Von-Frey
- J-5 : test de pression de la patte
- J-4 à J-1 : Injections préopératoires de mémantine ou NaCl (1 fois/jour) pour les rats du groupe devant subir une ligature du nerf spinal
- J0 : Ligature du nerf spinal (SNL)
- J0 à J2 : injections postopératoires de mémantine ou NaCl (1 fois/jour) pour les rats ayant subi une ligature du nerf spinal, ainsi que pour les rats du groupe contrôle (Sham).
- J7 : test Y-Maze
- J8 : test de Von-Frey
- J9 : test de pression de la patte
- J14 : test de Von-Frey
- J15 : test de pression de la patte
- J16 : Sacrifice des animaux et prélèvements de moelle épinière

### 6.1.3 Effets de la mémantine au niveau moléculaire :

Les effets de la mémantine sont également évalués au niveau de l'expression de la phosphorylation de la tyrosine 1472 de la sous-unité NR2B du récepteur NMDA au niveau spinal par la technique du western-blot.

Les rats sont sacrifiés par décapitation. Les renflements lombaires (L4-L6) sont prélevés et soumis à une lyse à 4°C dans 400 µL de tampon d'arrêt (50 mM Hepes, pH 7.5, contenant 150 mM NaCl, 10 mM EDTA, 10 mM Na₂P₂O₇, 2 mM Na₃VO₄, 100 mM NaF 1 % Triton, 0.5 mM PMSF, 100 UI/mL Iniprol et 20 µM Leupeptine.

Après sonication, deux centrifugations à 14.000 rpm à 4°C permettent de récupérer les protéines du cytoplasme. Le surnageant est récupéré et les protéines sont dosées par colorimétrie (BC Assay UP40840A®, Interchim). Elles sont dénaturées à 100°C pendant 5 min dans un tampon Tris 100 mM, pH 6.8, contenant 6 % SDS, 20 % Glycérol et 20 % bromophénol/β-mercaptoéthanol. Une électrophorèse permet de séparer les protéines sur gel de polyacrylamide. La migration s'effectue à 90 mA pendant 2h dans le tampon suivant : Tris 50 mM, pH 7.4, contenant 380 mM Glycine et 7 mM SDS. Les protéines sont ensuite transférées sur une membrane de nitrocellulose (Millipore) pendant 3h dans un tampon Tris 25 mM, pH 8.3, contenant 190 mM Glycine, 20 % méthanol (pourcentage volumique). Les protocoles ont été réalisés selon les recommandations du fournisseur et diffèrent en fonction de l'anticorps considéré.

Après blocage des sites non spécifiques avec une solution contenant 5 % de lait en poudre dénué de matière grasse, la membrane est incubée toute la nuit à 4°C sous agitation avec l'anticorps d'intérêt dilué dans la solution de blocage, à savoir un anticorps anti-pTyr¹⁴⁷²NR2B reconnaissant NR2B phosphorylée sur la tyrosine 1472 (dilution volumique = 1/1000, Millipore).

La membrane est ensuite hybridée 1h sous agitation avec un anticorps couplé à la péroxydase (dilution volumique = 1/10 000, Pierce) dilué dans le tampon de blocage.

La révélation est effectuée par chimioluminescence (Super Signal® West Pico Chimioluminescent Substrate, Pierce). L'analyse densitométrique des membranes est effectuée à l'aide d'un analyseur d'images (Chemidoc™, XRS Biorad) et d'un logiciel informatique (Image Lab™ Software).

Les données ont été exprimées par le ratio : intensité des bandes correspondant à la forme phosphorylée de la protéine d'intérêt (ici, pTyr¹⁴⁷²NR2B)/intensité des bandes correspondant à la forme totale (phosphorylée et non phosphorylée) de cette même protéine. Les résultats sont exprimés en % par rapport au contrôle, le contrôle étant la moyenne des valeurs obtenues sur au moins trois moelles épinières provenant d'animaux ayant reçu le véhicule.

### 6.1.4 Résultats:

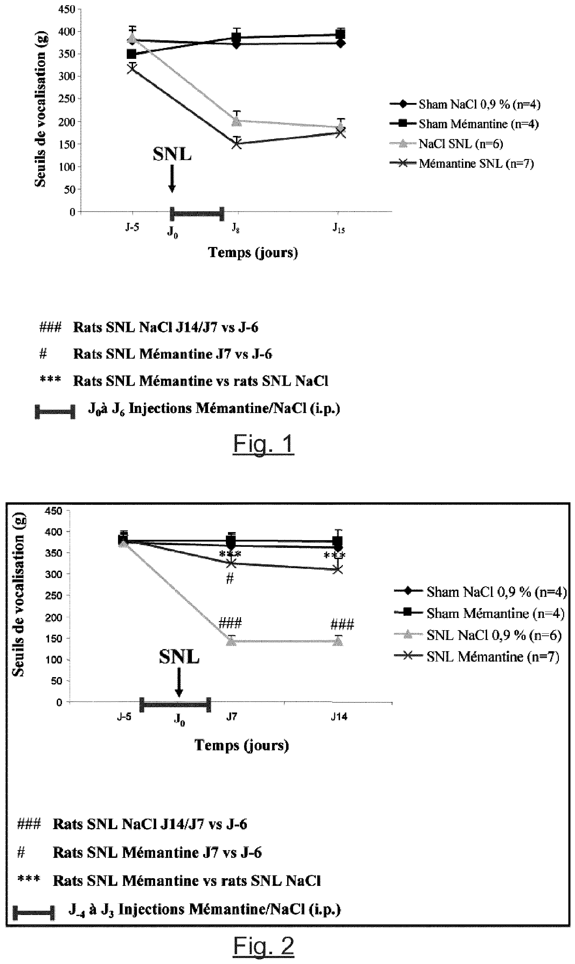

Les figures 1 et 2 illustrent les effets de l'administration de mémantine sur l'hyperalgésie mécanique, évaluée grâce au test de Randall et Sellito. La figure 1 présente les résultats obtenus pour les rats n'ayant reçu que les injections postopératoires soit de mémantine, soit du véhicule (à savoir, la solution saline de NaCl 0,9%). La figure 2 présente les résultats obtenus sur les rats ayant reçu soit le véhicule, soit la mémantine pendant les 4 jours précédents l'opération et pendant les 3 jours suivant l'opération. Selon ces deux figures, on constate que la mémantine seule n'influence en rien le comportement des rats non opérés par rapport aux rats non opérés ayant reçu le véhicule. Suite à la ligature, les rats présentent des signes évidents d'hyperalgésie. En relation avec la figure 1, l'injection postopératoire de mémantine ne suffit pas à rétablir un seuil de vocalisation normal entre les rats opérés et les rats sham. En revanche, les rats ayant reçu de la mémantine de manière préventive présentent un seuil de vocalisation très proche de ceux des rats non opérés, comme on peut le constater sur la figure 2. L'injection préventive de mémantine réussit donc à limiter l'hyperalgésie mécanique, suite à une douleur neuropathique.

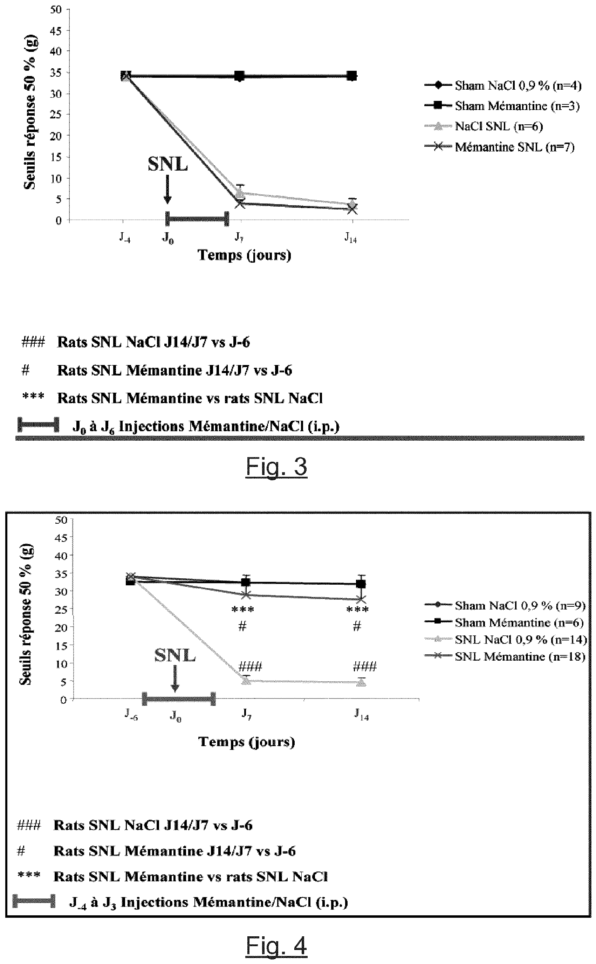

Les figures 3 et 4 illustrent les effets de l'administration de mémantine sur l'allodynie mécanique, évaluée grâce au test de Von Frey. La figure 3 présente les résultats obtenus pour les rats n'ayant reçu que les injections postopératoires soit de mémantine, soit du véhicule (à savoir, la solution saline de NaCl 0,9%). La figure 4 présente les résultats obtenus sur les rats ayant reçu soit le véhicule, soit la mémantine pendant les 4 jours précédents l'opération et pendant les 3 jours suivant l'opération. Selon ces deux figures, on constate que la mémantine seule n'influence en rien le comportement des rats non opérés par rapport aux rats non opérés ayant reçu le véhicule, les lignes se confondent sur les graphiques de ces deux figures. Suite à la ligature, les rats présentent des signes évidents d'allodynie. L'injection de mémantine uniquement à titre postopératoire ne suffit pas à rétablir le comportement normal des rats, comme il est possible de le constater sur la figure 3. En revanche, l'administration prophylactique de mémantine chez les rats opérés permet de rétablir un seuil de réponse quasi-identique à celui des rats non opérés. L'injection de véhicule ne permet pas d'obtenir le même résultat. Il est donc patent que l'administration prophylactique permet de supprimer la douleur neuropathique, ou du moins de limiter le développement de l'allodynie.

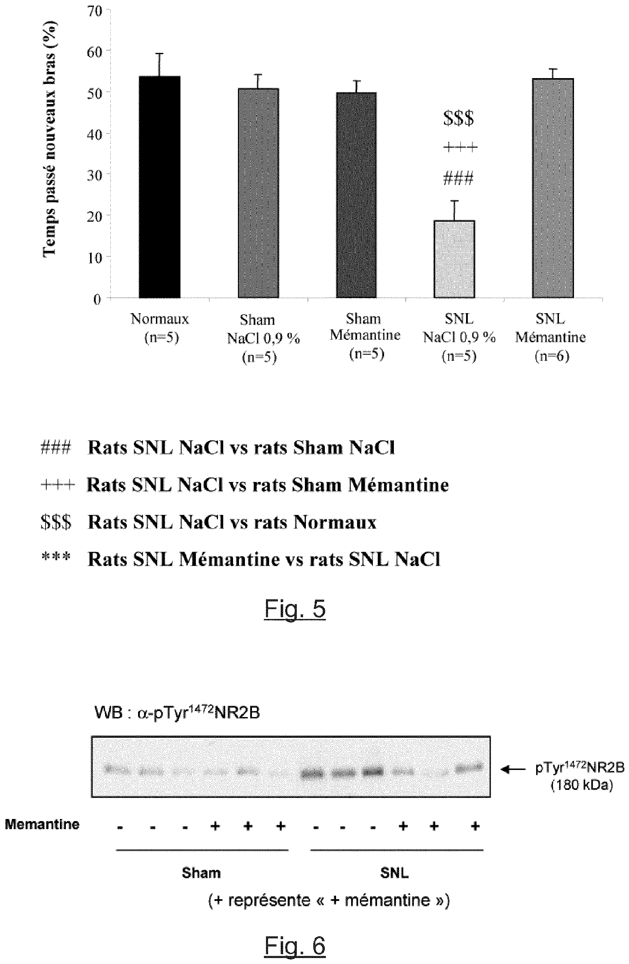

La figure 5 représente les résultats du test de Y-Maze, à savoir le temps passé à explorer les bras de la chambre (en pourcentage, par rapport au temps total passé dans la chambre). Les résultats sont présentés pour des rats normaux, n'ayant reçu aucun traitement et n'ayant subi aucune opération, pour les rats ayant reçu une injection pendant les 4 jours précédents leur opération et les 3 jours suivants leur opérations, que ce soit de la mémantine ou le véhicule et enfin pour les rats ayant reçu une injection de mémantine ou de véhicule pendant une semaine, sans subir de ligature.

On constate que l'injection de véhicule ou de mémantine ne modifie pas de manière significative la capacité d'exploration du rat par rapport aux rats normaux. La ligature du nerf spinal altère considérablement cette capacité, comme on peut le constater pour les rats SNL n'ayant reçu que le véhicule. Toutefois, cette capacité est rétablie pour les rats ayant reçu de la mémantine pendant les 4 jours précédents leur opération.

Après sacrifice des rats, la phosphorylation du résidu Tyrosine 1472 de la sous-unité NR2B est déterminée par Western Blot. La figure 6 représente une photographie de la membrane de Western, après révélation des bandes. On peut constater que, dans le groupe des rats Sham, la mémantine ne diminue pas de manière significative la phosphorylation du résidu Tyrosine 1472. En revanche, les rats traités de manière préventive par la mémantine présentent un taux de phosphorylation du résidu Tyrosine 1472 qui équivaut d'une part à celui des rats Sham non traités, et qui est bien inférieur d'autre part à celui des rats opérés et n'ayant pas été traités par la mémantine.

En conclusion, l'administration de mémantine avant une opération permet de prévenir l'apparition de la douleur neuropathique et de ses principaux symptômes, à savoir hyperalgésie, allodynie et troubles cognitifs, de manière plus efficace que l'administration de mémantine peropératoire et postopératoire.

Ce résultat peut être rapproché, sur le plan moléculaire, de l'inhibition de la phosphorylation du résidu Tyrosine 1472 de la sous unité NR2B des récepteurs NMDA. Il nous est donc permis de conclure que l'inhibition de la phosphorylation de ce résidu permet d'éviter le développement de la douleur neuropathique et des symptômes associés.

### 6.2 Exemple de protocole d'administration de la mémantine chez une patiente devant subir une opération induisant une douleur neuropathique : la mastectomie (tumorectomie et curage axillaire).

Chez une patiente devant subir une mastectomie pour cancer du sein, on administre de la mémantine, sous forme de chlorhydrate de mémantine, quotidiennement et pendant les 28 jours précédents son opération selon le régime suivant :
- Jour -28 à Jour -22 inclus : 5 mg/jour de chlorhydrate de mémantine,
- Jour -21 à Jour - 15 inclus : 10 mg/jour de chlorhydrate de mémantine,
- Jour -14 à jour -8 inclus : 15 mg/jour de chlorhydrate de mémantine,
- Jour -7 à jour 0 inclus : 20 mg/jour de chlorhydrate de mémantine.

Une dose de 20 mg de chlorhydrate de mémantine est également administrée à la patiente le jour de son opération (Jour 0), afin de maintenir le blocage des récepteurs NMDA par la mémantine.

La dose de 20 mg/jour de chlorhydrate de mémantine est maintenue pendant les deux semaines qui suivent l'opération, afin de limiter la survenue de la douleur neuropathique chez la patiente opérée.

Le chlorhydrate de mémantine est administré sous forme de comprimé pelliculé à avaler le matin, pendant ou en dehors des repas.

La douleur de la patiente peut ensuite être évaluée selon toute méthode usuelle : échelle numérique graduée de 1 à 10 (10 étant le stade maximal de la douleur), questionnaire d'évaluation du sommeil de Leeds (QESL), questionnaire de la qualité de vie SF36, questionnaire concis sur les douleurs (QCD), questionnaire sur la douleur neuropathique DN4, questionnaire de la douleur de Saint-Antoine (QDSA), échelle d'anxiété dépression (HAD), auto-questionnaire NPSI, le test cognitif du trail making test A et B, le test neuropsychologique DSST (Digit Symbol Substitution Test). L'évaluation de la quantité d'antalgiques ingérée par la patiente après son opération peut également être utilisée pour évaluer la douleur neuropathique.

### 6.3 Administration d'une composition selon l'invention chez des patientes devant subir une opération induisant une douleur neuropathique : la mastectomie (tumorectomie et curage axillaire) suite à un cancer du sein.

Un essai clinique de phase 2 portant sur le développement et l'évaluation de la douleur neuropathique est mené chez des patientes devant subir une mastectomie et un curage axillaire. Les patientes ont été séparées de manière aléatoire en deux groupes :
- un groupe témoin ayant reçu du lactose comme placebo; et
- un groupe traité par du chlorhydrate de mémantine (EBIXA®).

Le protocole de l'essai clinique est détaillé dans le formulaire référencé par le NTC014536314 (http://clinicaltrials.gov). Brièvement, les patientes reçoivent soit du chlorhydrate de mémantine, soit le placebo selon le protocole suivant :
- pendant les 2 semaines précédant l'intervention chirurgicale, les patientes reçoivent une dose comprise entre 5 mg/jour et 20 mg/jour, la dose administrée étant augmentée de manière régulière de 5 mg/jour comme cela est recommandé par l'AMM du médicament EBIXA® afin d'atteindre la dose de 20 mg/jour le jour de l'opération chirurgicale;
- le jour de leur intervention, les patientes reçoivent une dose de 20 mg/jour, et
- pendant les 2 semaines suivant l'intervention chirurgicale, les patientes reçoivent une dose constante de 20 mg/jour.

Lors des deux semaines précédant l'opération, les patientes ont reçu une dose croissante par palier, de 5 mg/jour de la composition à base de mémantine ou du placebo.

La douleur de la patiente a été évaluée en demandant à la patiente d'évaluer sa propre douleur sur une échelle numérique graduée de 1 à 10 (10 étant le stade maximal de la douleur) pendant les deux semaines suivant l'opération et pendant 3 mois suivants l'opération de la patiente. Cette méthode d'évaluation de la douleur est communément utilisée par les cliniciens. Le développement de douleur neuropathique est également évalué par le questionnaire sur la douleur neuropathique DN4 et le questionnaire de la douleur de Saint-Antoine (QDSA). D'autres méthodes d'évaluation ont également été utilisées comme le questionnaire d'évaluation du sommeil de Leeds (QESL), le questionnaire de la qualité de vie SF36, le questionnaire concis sur les douleurs (QCD), l'échelle d'anxiété dépression (HAD), l'auto-questionnaire NPSI, le test cognitif du trail making test A et B, et le test neuropsychologique DSST (Digit Symbol Substitution Test).

Les patientes ont été interrogées 15 jour avant leur intervention (D-15 jours), à leur entrée dans l'essai clinique, le jour de leur intervention (D0), puis 5 jours (D + 5jours), 15 jours (D + 15 jours) et 3 mois après l'opération (D + 3 mois).

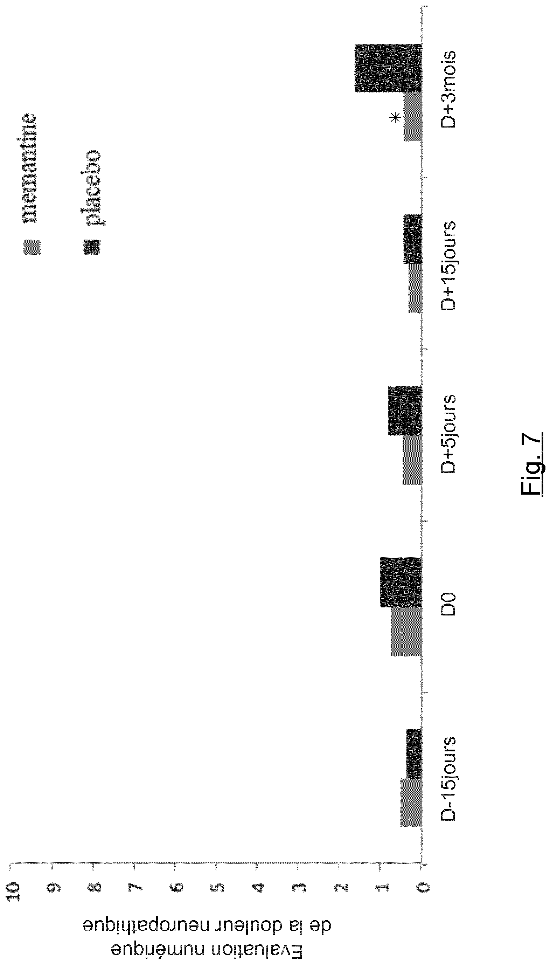

La figure 7 est un graphique présentant les résultats préliminaires obtenus sur les quinze premières patientes ayant participé à cet essai clinique (6 patientes ayant reçu la composition placébo et 7 patientes ayant reçu la composition à base de chlorhydrate de mémantine), et dont la douleur a été évaluée par la méthode d'auto-évaluation sur une échelle de 1 à 10. Le jour de l'opération est indiqué par D sur le graphique. L'étoile indique une signifiance statistique (p<0.05).

Comme cela est montré à la figure 7, les patientes ayant reçu le placebo présentent un niveau de douleur plus élevé par rapport aux patientes ayant reçu de la mémantine. Trois mois après l'intervention, la différence entre les patientes traitées de manière préventive par la mémantine et les patientes traitées selon le même protocole est significative, bien que la dernière administration ait eu lieu 2 semaines après l'opération. En effet, les patientes ayant reçu la composition à base de chlorhydrate de mémantine de manière préventive au sens de l'invention, ne présentent pas de douleur (compris entre 0 et 0,5) tandis que les patientes ayant reçu le placebo continue de présenter des niveaux de douleur en moyenne de 2. Ces résultats sont corrélés par les résultats obtenus avec les autres méthodes d'évaluation.

### 7. Conclusion

La composition selon l'invention, administrée de manière préventive c'est à dire un à plusieurs jours avant le jour de l'opération chirurgicale, permet donc d'atténuer et de limiter durablement le développement de la douleur neuropathique chez le patient. Les inventeurs ont ainsi démontré que l'administration prophylactique de mémantine, c'est à dire avant l'opération et non uniquement au moment de l'opération, permet de limiter grandement le développement de la douleur neuropathique par la non-sensibilisation précoce des récepteurs NMDA impliqués dans ce type de douleur.

La présente invention permet donc de réduire, voire d'éviter, la souffrance des patients opérés de manière plus efficace. Jusqu'à présent, la mémantine était administrée aux patients au moment de leur opération, cette administration étant suivie après l'opération pour traiter leur douleur. En administrant la mémantine avant, pendant une durée comprise entre 1 et 42 jours avant l'opération, les inventeurs ont trouvé le moyen de limiter, voire d'éviter, le développement de la douleur neuropathique.

## Revendications

1. Composition analgésique contenant de la mémantine, **caractérisée en ce qu'**elle est administrée quotidiennement à un patient humain devant subir une opération chirurgicale, sur une période comprise entre 1 jour et 42 jours avant ladite opération chirurgicale, pour l'utilisation dans la prévention du développement de la douleur neuropathique post-opératoire chez ce même patient.

2. Composition pour l'utilisation selon la revendication 1 **caractérisée en ce qu'**elle est administrée pendant une période comprise entre 1 jour et 28 jours avant ladite opération du patient.

3. Composition pour l'utilisation selon l'une des revendications précédentes **caractérisée en ce que** l'administration préopératoire de ladite composition se fait par paliers successifs de doses croissantes.

4. Composition pour l'utilisation selon l'une des revendications précédentes **caractérisée en ce qu'**elle est administrée sous forme de chlorhydrate de mémantine à une dose comprise entre 5 mg/jour et 20 mg/jour.

5. Composition pour l'utilisation selon l'une des revendications précédentes **caractérisée en ce qu'**elle est administrée par voie orale sous forme de comprimé pelliculé ou de solution buvable.

6. Composition pour l'utilisation selon l'une des revendications 1 à 4 **caractérisée en ce qu'**elle est administrée par perfusion à une concentration comprise entre 5 mg/jour/kg et 20 mg/jour/kg.

7. Composition pour l'utilisation selon l'une des revendications précédentes **caractérisée en ce qu'**elle est destinée à être administrée à un patient devant subir une chirurgie pouvant entraîner des douleurs neuropathiques, ladite chirurgie étant choisie parmi l'ablation d'un membre, d'une partie d'un membre, d'un organe ou d'une partie d'un organe.

## Patentansprüche

1. Analgetische Zusammensetzung, enthaltend Memantin, **dadurch gekennzeichnet, dass** sie täglich an einen menschlichen Patienten, der sich einer chirurgischen Operation unterziehen muss, über einen Zeitraum im Bereich zwischen 1 Tag und 42 Tagen vor der chirurgischen Operation verabreicht wird, zur Verwendung bei der Prävention der Entwicklung von postoperativen neuropathischen Schmerzen bei ebendiesem Patienten.

2. Zusammensetzung zur Verwendung nach Anspruch 1, **dadurch gekennzeichnet, dass** sie über einen Zeitraum im Bereich zwischen 1 Tag und 28 Tagen vor der Operation des Patienten verabreicht wird.

3. Zusammensetzung zur Verwendung nach einem der vorhergehenden Ansprüche, **dadurch gekennzeichnet, dass** die präoperative Verabreichung der Zusammensetzung schrittweise mit steigenden Dosen erfolgt.

4. Zusammensetzung zur Verwendung nach einem der vorhergehenden Ansprüche, **dadurch gekennzeichnet, dass** sie in Form von Memantinhydrochlorid in einer Dosis im Bereich zwischen 5 mg/Tag und 20 mg/Tag verabreicht wird.

5. Zusammensetzung zur Verwendung nach einem der vorhergehenden Ansprüche, **dadurch gekennzeichnet, dass** sie oral in Form von Filmtabletten oder einer Trinklösung verabreicht wird.

6. Zusammensetzung zur Verwendung nach einem der Ansprüche 1 bis 4, **dadurch gekennzeichnet, dass** sie durch Perfusion in einer Konzentration im Bereich zwischen 5 mg/Tag/kg und 20 mg/Tag/kg verabreicht wird.

7. Zusammensetzung zur Verwendung nach einem der vorhergehenden Ansprüche, **dadurch gekennzeichnet, dass** sie dazu bestimmt ist, an einen Patienten verabreicht zu werden, der sich einem chirurgischen Eingriff unterziehen muss, der zu neuropathischen Schmerzen führen kann, wobei der chirurgische Eingriff ausgewählt ist aus der Ablation eines Körperteils, eines Teils eines Körperteils, eines Organs oder eines Teils eines Organs.

## Claims

1. Analgesic composition containing memantine, **characterized in that** it is administered daily, to a human patient who is to undergo a surgical operation, for a period ranging from one to 42 days before the surgical operation, for the use in the prevention of the development of post-operative neuropathic pain in this patient.

2. Composition for use according to claim 1, **characterized in that** it is administered for a period ranging from 1 day to 28 days before said surgical operation of the patient.

3. Composition for use according to any one of the preceding claims, **characterized in that** the pre-operative administration of said composition is done in successive stages of increasing doses.

4. Composition for use according to any one of the preceding claims, **characterized in that** it is administered in memantine hydrochloride form in a dose ranging from 5 mg/day to 20 mg/day.

5. Composition for use according to any one of the preceding claims, **characterized in that** it is administered orally, in the form of a coated pill or a drinkable solution.

6. Composition for use according to one of the claims 1 to 4 **characterized in that** it is administered by perfusion in a concentration ranging from 5 mg/day/kg to 20 mg/day/kg.

7. Composition according to any one of the preceding claims, **characterized in that** it is intended for administration to a patient who has to undergo surgery that can cause neuropathic pain, said surgery being one of the following: ablation of a limb, of a part of a limb, an organ or a part of an organ.
